# EUROPÄISCHE PATENTSCHRIFT

(11) **EP 1 965 911 B1**
(45) Veröffentlichungstag und Bekanntmachung des Hinweises auf die Patenterteilung: **14.08.2013**
(21) Anmeldenummer: 06818776.4
(22) Anmeldetag: 23.11.2006
(51) Int. Cl.: B01J 23/881, B01J 35/02, B01J 37/02, B01J 37/08, C07C 45/38

(54) **SCHALENKATALYSATOR, INSBESONDERE ZUR OXIDATION VON METHANOL ZU FORMALDEHYD SOWIE VERFAHREN ZU DESSEN HERSTELLUNG**
SHELL CATALYST, IN PARTICULAR FOR OXIDATION OF METHANOL TO FORMALDEHYDE, AND ALSO METHOD FOR PRODUCTION THEREOF
CATALYSEUR SOUS FORME DE COQUE, NOTAMMENT POUR L'OXYDATION DE METHANOL EN FORMALDEHYDE, ET SON PROCEDE DE PRODUCTION

(30) Priorität: 23.11.2005 DE 102005055827; 23.11.2005 EP 05025497
(43) Veröffentlichungstag der Anmeldung: 10.09.2008
(73) Patentinhaber: Süd-Chemie IP GmbH & Co. KG, 80333 München (DE)
(72) Erfinder: GÜCKEL, Christian, Paramus, NJ 07652 (US); WANNINGER, Klaus, 83059 Kolbermoor (DE); ESTENFELDER, Marvin, I-28100 Novara (IT); FISCHER, Claudia, 83043 Bad Aibling (DE); DÜRR, Uwe, 90765 Fürth (DE)
(74) Vertreter: Stolmár, Matthias
(86) Internationale Anmeldenummer: PCT/EP2006/011256
(87) Internationale Veröffentlichungsnummer: WO 2007/059974

(56) Entgegenhaltungen:
- EP-A- 0 184 790
- EP-A- 1 108 470
- EP-A2- 0 068 192
- WO-A-98/23371
- WO-A-99/61433
- WO-A-99/62637
- WO-A-2004/035473
- WO-A-2005/037427
- WO-A1-2005/030388
- DE-A1-102004 014 918
- US-A- 5 217 936

## Beschreibung

Die vorliegende Erfindung betrifft einen Schalenkatalysator, im speziellen einen beschichteten Katalysator (Beschichtungskatalysator), insbesondere zur Oxidation von Methanol zu Formaldehyd, der auf einem inerten, vorzugsweise im wesentlichen unporösen Trägerkörper eine Beschichtung aus einer Zusammensetzung aufweist, die neben einer aktiven Masse eine Haftungsvermittlerzusammensetzung enthält, die mindestens einen organischen Binder und mindestens eine weitere, zumindest teilweise anorganische haftungsvermittelnde Komponente, insbesondere eine Solkomponente umfasst.

Molybdän-Eisen-Katalysatoren zur partiellen Oxidation von Methanol zu Formaldehyd sind schon seit längerer Zeit bekannt.

Die Atomverhältnisse zwischen Molybdän und Eisen können bei diesen bekannten Katalysatoren variieren. Ferner kann ein Teil der aktiven Komponenten teilweise durch Titan, Antimon, Zinn, Nickel, Chrom, Cer, Aluminium, Calcium, Magnesium, Vanadium, Niob, Silber und/oder Mangan ersetzt sein. Derartige zum Beispiel Titan enthaltende Katalysatoren sind beispielsweise aus der US 3,978,136 für die partielle Oxidation von Methanol zu Formaldehyd.bekannt. Hierbei handelt es sich aber im Gegensatz zu dem erfindungsgemäßen Katalysator nicht um einen gecoateten Katalysator.

Unter gecoateten (beschichteten) Katalysatoren, auch Coat- oder Beschichtungskatalysatoren genannt, versteht man Katalysatoren, die durch Beschichten eines (unporösen) Trägerkörpers mit einer porösen Schicht der eigentlichen aktiven Masse entstehen.

Im Unterschied dazu werden bei den Imprägnierverfahren die katalytisch aktiven Zentren (häufig Edelmetalle, wie Pd, Pt, Au, Ag etc.) als Lösung dispers auf einen porösen Träger (häufig: SiO₂, Al₂O₃, TiO₂, ZrO₂, Carbon, Nb₂O₅ etc.) aufgebracht. Bei den durch das Imprägnierverfahren hergestellten Katalysatoren bestehen zumeist chemisch-physikalische Wechselwirkungen zwischen Träger und aktiven Zentren, die entscheidenden Einfluss auf das katalytische Geschehen nehmen. Hingegen dient bei den Coatkatalysatoren der Trägerkörper lediglich der Formgebung ("structural support"). Im Gegensatz zu imprägnierten Katalysatoren, bei denen die aktiven Elemente dispers in dem porösen Träger - gegebenenfalls auch in einer im Träger befindlichen äußeren Schale (= Schalenkatalysator) - verteilt sind, ist beim Coatkatalysator der (unporöse bzw. im wesentlichen unporöse) Trägerkörper von der aktiven Masse umhüllt.

In der US 3,975,302 wird ein nach dem Imprägnierverfahren hergestellter Fe/Mo-Katalysator für die Oxidation von Methanol zu Formaldehyd beschrieben. Demnach werden Eisen und Molybdän als MoO₄²⁻- und Fe³⁺- Salze in Wasser gelöst und dann auf einen porösen Träger mit einer BET-Oberfläche von 1 bis 20 m²/g imprägniert.

Auch in der US 4,181,629 wird ein Katalysator für die Oxidation von Methanol zu Formaldehyd beschrieben, der durch Imprägnieren im Fließbettverfahren hergestellt wird.

Die EP-A-0 068 192 betrifft einen abriebfesten Schalenkatalysator, wobei als bevorzugtes Bindemittel in der Schalenzusammensetzung Glucose- oder Harnstoff in wässriger Lösung angegeben sind.

Die EP-A-0 294 775 betrifft ein Verfahren zur Herstellung eines Schalenkatalysators durch Aufsprühen des katalytisch aktiven Materials auf bewegte Trägerteilchen bei Temperaturen von 100 bis 600 °C.

In der WO 98/23371 wird ein Schalenkatalysator zur Herstellung von Essigsäure durch Gasphasenoxidation ungesättigter C₄-Kohlenstoffe beschrieben. Der Katalysator umfasst einen inerten unporösen Trägerkörper, auf dessen Oberfläche eine katalytisch aktive Mischoxidmasse aufgebracht ist. Diese Masse umfasst (a) ein oder mehrere Oxide aus der Gruppe Titanoxid, Zirkondioxid, Zinndioxid, Aluminiumoxid, sowie (b) bezogen auf das Gewicht der Komponente (a) und pro m²/g spezifischer Oberfläche der Komponente (a) 0,1 bis 1,5 Gew.-% Vanadiumpentoxid. Ein Teil des Vanadiumpentoxids, vorzugsweise 10 bis 90 Gew.-%, kann durch ein oder mehrere Oxide von Molybdän, Chrom und Antimon ersetzt sein. Gegebenenfalls können als zusätzliche Komponente (b) noch ein oder mehrere Oxide von Alkalimetallen, Elementen der 5. und 6. Hauptgruppe des Periodensystems der Elemente und der Übergangsmetalle enthalten sein. Genannt wird unter anderen auch Eisen. Der Anteil dieser Dotierstoffe beträgt 0,05 bis 15 Gew.-% berechnet als Oxid und bezogen auf das Gesamtgewicht der Komponente (b). Die katalytisch aktive Mischoxidmasse kann ggf. noch 10 bis 50 Gew.-%, bezogen auf das Gesamtgewicht der katalytisch aktiven Mischoxidmasse, inerte Verdünnungsmittel, wie Siliziumdioxid oder Siliciumcarbid enthalten. Als Bindemittel werden organische Bindemittel eingesetzt. Zur Herstellung der Katalysatoren werden die Aktivkomponenten unter Zusatz von Wasser vermahlen. Anschließend wird eine Copolymerdispersion aus Vinylacetat und Vinyllaurat zugegeben. Die fertige Suspension wird unter Verdampfen des Wassers auf Steatitkugeln aufgebracht. Es wird in den Beispielen kein Katalysator beschrieben, der gleichzeitig Molybdän und Eisen enthält.

In der WO 99/62637 wird ein Verfahren zur Herstellung von Schalenkatalysatoren für die katalytische Gasphasenoxidation von aromatischen Kohlenwasserstoffen beschrieben. Der Katalysator besteht aus einem Trägerkern und darauf schalenförmig aufgebrachten katalytisch wirksamen Metalloxiden. Der Katalysator wird hergestellt, indem eine wässrige Aktivmassensuspension auf das heiße Trägermaterial aufgesprüht wird. Die Aktivmassensuspension enthält ein organisches Bindemittel. Dieses wird aus (A) einem Polymerisat gebildet, das hergestellt wird aus (a) 5 bis 100 Gew.-% Monomeren in Form von ethylenisch ungesättigten Säureanhydriden oder ethylenisch ungesättigten Dicarbonsäuren, deren Carboxylgruppen ein Anhydrid bilden können, und (b) 0 bis 95 Gew.-% monoethylenisch ungesättigten Monomeren, mit der Maßgabe, dass die Monomeren (a) und (b) durchschnittlich höchstens 6 Kohlenstoffatome aufweisen, die nicht mit Sauerstoff enthaltenden Gruppen funktionalisiert sind, und (B) einem Alkanolamin mit mindestens 2 OH-Gruppen, höchstens 2 Stickstoffatomen und höchstens 8 C-Atomen, wobei das Gewichtsverhältnis (A) : (B) 1 : 0,05 bis 1 : 1 beträgt. Als katalytisch aktiver Bestandteil der katalytisch aktiven Masse dient im allgemeinen neben Titandioxid in seiner Anatasmodifikation Vanadiumpentoxid. Neben diesen Hauptbestandteilen kann eine Vielzahl anderer oxidischer Verbindungen enthalten sein, die als Promotoren die Aktivität und Selektivität des Katalysators beeinflussen. Als beispielhafte Promotoren werden die Alkalimetalloxide, Thallium(I)oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid und Phosphorpentoxid genannt. Die Suspension der Aktivmasse wird durch Versprühen auf den Träger aufgetragen. Dies kann beispielsweise in einer Dragiertrommel oder in einem Wirbelbettbeschichter erfolgen. In den Beispielen wird kein Katalysator beschrieben, der gleichzeitig Molybdän und Eisen besitzt. Neben dem organischen Bindemittel wird kein weiteres Bindemittel verwendet.

In der US 5,217,936 wird ein Katalysator für die Herstellung von Aldehyden aus den entsprechenden Alkoholen, insbesondere von Formaldehyd aus Methanol beschrieben, wobei die katalytisch aktive Masse auf einen monolithischen Träger aufgetragen ist. Die aktive Masse kann neben Molybdänoxid noch Oxide von Chrom, Vanadium, Aluminium, Eisen, Wolfram, Mangan sowie deren Mischungen enthalten. Um die Bindung der aktiven Masse zum monolithischen Träger zu verbessern, kann die aktive Masse ein Bindemittel enthalten. Als geeignetes Bindemittel wird Siliziumdioxid sowie Titandioxid angegeben. Die Verwendung von organischen Bindemitteln wird nicht beschrieben.

In der EP 1 108 470 A1 werden Trägerkatalysatoren beschrieben, die sich für die Gasphasenoxidation von Kohlenwasserstoffen eignen. Die Trägerkatalysatoren bestehen aus einer aktiven Masse, die auf einem inerten Trägerkörper aufgetragen ist. Der Trägerkörper hat die Form eines Rings, in den ein oder mehrere Kerben an der oberen und/oder unteren Flachseite des Ringes eingebracht sind.

In der WO 99/61433 wird ein Verfahren zur Herstellung von Phthalsäureanhydrid durch katalytische Gasphasenoxidation von Xylol oder Naphthalin beschrieben, wobei ein Reaktor verwendet wird, der mindestens drei übereinander angeordnete Schichten eines Schalenkatalysators umfasst, die von der Gaseintritts- zur Gasaustrittsseite des Reaktors zunehmende Aktivität aufweisen. Die aktive Masse wird aus Oxiden des Vanadiums und Antimons, Phosphors, der Alkalimetalle sowie des Titans gebildet. Die Aktivität wird durch die Menge der auf den Trägerkörpern aufgetragenen Aktivmasse sowie durch den Gehalt an Alkalimetall in der aktiven Masse eingestellt. Die Herstellung des Katalysators erfolgt, indem eine wässrige Suspension der aktiven Masse auf inerte Trägerkörper aufgesprüht wird, beispielsweise in einer Dragiertrommel oder in einem Wirbelbettbeschichter. Um Verluste an aktiver Masse beim Beschichten zu verringern, kann die Suspension ein organisches Polymer als Bindemittel enthalten. Die Verwendung anorganischer Bindemittel wird nicht beschrieben.

In der DE 10 2004 014 918 wird ein Katalysator mit einer Silber-Vanadiumoxidphase und einer Promotorphase auf Basis von Titandioxid und Vanadiumpentoxid beschrieben, welcher für die Herstellung von Aldehyden, Carbonsäuren und Carbonsäureanhydriden aus aromatischen oder heteroaromatischen Kohlenwasserstoffen durch Gasphasenoxidation geeignet ist. Der Katalysator ist bevorzugt als Schalenkatalysator ausgebildet, wobei die beiden Phasen als konzentrische Schalen auf einem inerten Träger angeordnet sind. Zur Herstellung des Schalenkatalysators wird eine wässrige Aufschlämmung der Aktivmaterialien mit Hilfe einer Dragiertrommel oder eines Wirbelbettbeschichters auf die Trägerkörper aufgesprüht. Der Aufschlämmung kann ein Bindemittel beigegeben werden. Als geeignete Bindemittel werden höhere Alkohole, DMF, cyclische Harnstoffe, usw., sowie polymere Bindemittel, wie Vinylacetat/Vinyllaurat-Copolymere genannt. Die Verwendung anorganischer Bindemittel wird nicht beschrieben.

In der WO 2005/037427 wird eine Katalysatorschüttung aus einer physikalischen Mischung von katalytisch aktiven und katalytisch inaktiven Formkörpern beschrieben, wobei die katalytisch inaktiven Formkörper an den äußeren Reibungsflächen abgerundete Kanten aufweisen. Wird die Katalysatorschüttung für die Oxidation von Methanol zu Formaldehyd eingesetzt, können als katalytisch aktive Masse beispielsweise Eisenmolybdate eingesetzt werden.

In der EP 0 184 790 wird ein geformter Katalysator für heterogen katalysierte Reaktionen beschrieben, der aus einem zu Hohlzylindern geformten inerten Trägermaterial besteht, auf den ein katalytisch aktives Material aufgebracht ist. Der Außendurchmesser der Hohlzylinder beträgt 3 bis 20 mm und der Innendurchmesser beträgt das 0,1- bis 0,7-fache des Außendurchmessers. Die Stirnfläche des Hohlzylinders ist so gekrümmt, dass der Radius der Krümmung das 0,4- bis 5-fache des Außendurchmessers beträgt. Die Katalysatoren eignen sich insbesondere für partielle Oxidationen in der Gasphase.

In der EP 0 068 192 wird ein Verfahren zur Herstellung abriebfester Schalenkatalysatoren beschrieben, welche u.a. für Gasphasenoxidationen in der organischen Chemie eingesetzt werden können. Der Schalenkatalysator besteht aus einem inerten Träger und einer diesen umgebenden Schale aus Katalysatormaterial. Zur Herstellung des Katalysators wird eine Suspension des Ausgangsmaterials für die Schale unter Teilabzug des suspensionsmittels durch einen Gasstrom auf eine bewegte Schüttung des Trägers aufgesprüht und das Rohmaterial dann getrocknet und getempert. Hierzu wird die Trägerschüttung mechanisch unter Auflockerung durch einen von unten eingeblasenen Gasstrom bewegt. Die ein Bindemittel und gegebenenfalls Porenbildner enthaltende Katalysatorvorstufe wird in ansteigender Menge von oben auf die Schüttung aufgesprüht, wobei das Mengenverhältnis zwischen aufgesprühtem und abgezogenem Suspensionsmittel etwa konstant bleibt. Der Wärmeausdehnungskoeffizient der Vorstufe als trockenes Pulver darf dabei höchstens um 15 % von dem des Trägers abweichen. Die aufgebrachte Schale wird durch Fortsetzen der mechanischen und fluidisierenden Mischbewegung verdichtet, das Gut dann im weiterströmenden Gas getrocknet und gegebenenfalls nach Zersetzen eines zugegebenen Porenbildners, getempert.

Als nachteilig an den bekannten Katalysatoren wird beschrieben, dass bei dickeren Schalen, also Schalen, deren Gewichtsmenge, bezogen auf den Katalysator, 20 % übersteigt, die Abrieb- und Stoßfestigkeit der Schale für den Einsatz in großtechnischen Festbettreaktoren nicht vollständig befriedigt.

Insbesondere bei Schalenkatalysatoren, welche mit konventionellen Dragierkesseln oder Drehtellern gefertigt werden, welche nur ein Überleiten eines trocknenden Gasstroms über das bewegte Gut gestatten, zeige sich eine Neigung zum Abplatzen der Schale bei Einwirkung von Temperaturgradienten.

Um die Schale fest auf dem inerten Kern zu verankern, werden verschiedene Maßnahmen vorgeschlagen;

So ist der inerte Träger oberflächlich rau ausgeführt, damit die Haftfestigkeit der Schale durch eine Tiefenverankerung des katalytisch aktiven Materials im Träger erhöht und ein gleichmäßiger Auftrag auf der gesamten Trägeroberfläche ermöglicht wird.

Ferner kann der katalytisch aktiven Masse ein Bindemittel zugegeben werden. Als besonders vorteilhaft sind Glucose oder Harnstoff angegeben.

Als wesentlich wird jedoch eine konstante Feuchte des Materials der Schale während ihres Auftrags beschrieben. Dazu wird die Menge an abgezogenem und aufgesprühtem Suspensionsmittel in einem weitgehend konstanten Verhältnis gehalten. Ferner soll der Wärmeausdehnungskoeffizient des Vorstufenpulvers durch eine Wärmebehandlung auf den des Trägers abgestimmt werden, sodass diese sich nicht um mehr als 15 % unterscheiden.

Es besteht jedoch ein Bedarf an verbesserten Katalysatoren zur Oxidation von Methanol zu Formaldehyd, die neben einer sehr guten Abriebfestigkeit auch eine gute Aktivität und Selektivität aufweisen. Aufgabe der vorliegenden Erfindung war es daher, verbesserte Katalysatoren, insbesondere zur Oxidation von Methanol zu Formaldehyd bereitzustellen, die eine besonders hohe Abriebfestigkeit aufweisen und die Nachteile des Standes der Technik vermeiden.

Diese Aufgabe wird gemäß Anspruch 1 gelöst durch einen Schalen- bzw. Beschichtungskatalysator in ungetemperter Form, insbesondere zur Oxidation von Methanol zu Formaldehyd, der auf einem inerten, vorzugsweise im wesentlichen unporösen Trägerkörper in Form eines Hohlzylinders mindestens eine Beschichtung aufweist, die vor dem Entfernen der organischen Anteile der Komponenten b) bzw. c) enthält:
a) Oxide oder in die entsprechenden Oxide überführbare Vorläuferverbindungen von Molybdän und Eisen, wobei das molare Verhältnis von Mo: Fe zwischen 1:1 und 5:1 liegt, sowie gegebenenfalls weitere metallische bzw. metalloxidische Komponenten oder in die entsprechenden Oxide überführbare Vorläuferverbindungen,
b) mindestens einen organischen Binder, wobei das organische Bindemittel ein Copolymer ausgewählt aus Vinylacetat/Vinyllaurat, Vinylacetat/Ethylen, Vinylacetat/Acrylat, Vinylacetat/Maleat, Styrol/Acrylat oder deren Gemischen ist,
c) und mindestens eine weitere haftungsvermittelnde Komponente ausgewählt aus der Gruppe bestehend aus SiO₂-Sol oder dessen Vorläufer, Al₂O₃-Sol oder dessen Vorläufer, ZrO₂-Sol oder dessen Vorläufer, TiO₂- Sol oder dessen Vorläufer, Ce₂O-sol, Wasserglas, MgO, Zement, monomeren, oligomeren oder polymeren Silanen, Alkoxysilanen, Aryloxysilanen, Acryloxysilanen, Aminosilanen, Siloxanen oder Silanolen,
wobei der Anteil der Komponente c) nach dem Entfernen der organischen Anteile der Komponenten b) und ggf. c) zwischen 0,01 und 30 Gew.-% beträgt, bezogen auf die katalytisch aktive Beschichtungszusammensetzung.

So wurde überraschend gefunden, dass ein Beschichtungs- bzw. Schalenkatalysator, bei dem die Beschichtungszusammensetzung des Trägerkörpers neben der (katalytisch) aktiven Masse, enthaltend Oxide oder in die entsprechenden Oxide überführbare Vorläuferverbindungen von Molybdän und Eisen (Komponente a)), eine Kombination aus mindestens einem organischen Binder, der beim Tempern bzw. Calcinieren entfernt (ausgebrannt) wird, und mindestens einer weiteren Komponente, ausgewählt aus der Gruppe SiO₂-Sol oder dessen Vorläufer, Al₂O₃-Sol oder dessen Vorläufer, ZrO₂-Sol oder dessen Vorläufer, TiO₂-Sol oder dessen Vorläufer, Wasserglas, MgO, Zement, monomeres, oligomeres oder polymeres Silan, Alkoxysilan, Aryloxysilan, Acryloxysilan, Aminosilan, Siloxan oder Silanole, enthält, eine besonders hohe Abriebfestigkeit zeigt. Dabei wurde unerwartet gefunden, dass nicht nur die ungetemperten, d.h. die organischen Teile der BeschichtungsZusammensetzung enthaltenden erfindungsgemäßen Beschichtungskatalysatoren besonders abriebfest sind, sondern nach dem Tempern bzw. Calcinieren der erfindungsgemäßen Katalysatoren weiterhin eine besonders hohe Abriebfestigkeit gegeben ist und eine für die Aktivität des Katalysators zur Oxidation von Methanol zu Formaldehyd besonders günstige Porosimetrie bereitgestellt wird. Dabei kann hierin der (noch nicht getemperte bzw. calcinierte) Katalysator, bei dem die organischen Anteile der Komponenten b) und c) noch nicht entfernt wurden, auch als Katalysator-Vorstufe bzw. Vorläufer-Katalysator angesehen werden. In dem fertigen Katalysator, wie er zur Oxidation von Methanol zu Formaldehyd eingesetzt wird, sind dann die organischen Anteile der Komponenten b) und c) entfernt, insbesondere durch Tempern bzw. Calcinieren.

Vereinfacht gesagt umfasst die Beschichtung in den erfindungsgemäßen Beschichtungskatalysatoren neben der aktiven Masse (Komponente a)) noch mindestens zwei als Haftungsvermittler wirkende Komponenten. Davon stellt mindestens eine Komponente einen organischen Binder dar (Komponente b)), der beim Tempern und Calcinieren des Katalysators entfernt werden kann. Die zweite Komponente (Komponente c)) weist zumindest einen anorganischen Anteil auf oder umfasst eine rein anorganische Verbindung, die nach dem Tempern bzw. Calcinieren des Beschichtungskatalysators Brücken zwischen den oxidischen Bestandteilen der aktiven Masse, d.h. den molybdän- bzw. eisenhaltigen Oxiden in vorteilhafter Weise bilden kann. Die anorganische Komponente bzw. deren anorganischer Anteil bildet, ohne dass die Erfindung auf die Richtigkeit des theoretischen Mechanismus beschränkt wäre, offenbar Verbindungen zwischen den Primärteilchen der Fe- bzw. Mo-Oxide, insbesondere des Eisenmolybdän-Mischoxids bzw. der anderen katalytisch aktiven Komponenten (a)). Die organische Komponente b) und, soweit vorhanden, die organischen Anteile der Komponente c) (siehe oben) ermöglichen dabei in enger Assoziation zu den Primärteilchen der katalytisch aktiven Komponente a), jedoch ohne deren Verbrückung durch die haftungsvermittelnde anorganischen Komponente bzw. deren anorganische Anteile zu beeinträchtigen, die Ausbildung von Poren und somit im fertigen Katalysator den optimalen Zutritt der Reaktanden zu den aktiven Zentren. Erfindungsgemäß wird als Komponente b) ein organischer Binder aus der vorher genannten Gruppe eingesetzt. Die Verwendung dieses organischen Binders hat den Vorteil, dass sich der Beschichtungsvorgang in einer Fließbettapparatur mit besonders geringen Sprühverlusten bewerkstelligen lässt. Zudem bildet sich ein vorteilhaftes Porengefüge nach dessen Entfernung aus.

Erfindungsgemäss umfasst der Beschichtungskatalysator zunächst einen inerten Trägerkörper. Hier können grundsätzlich alle dem Fachmann geläufigen inerten Trägerkörper verwendet werden. Vorzugsweise sollten die Trägerkörper jedoch im Wesentlichen unporös sein und eine BET-Oberflache (bestimmt nach DIN 66131) von weniger als etwa 1 m²/g aufweisen. Das Porenvolumen des inerten, im Wesentlichen unporösen Trägerkörpers soll vorzugsweise bei weniger als 0,1 ml/g, bestimmt gemäß DIN 66133, liegen. Die Materialdichte der Trägerkörper liegt vorzugsweise im Bereich von 2,0 bis 4,5 g/cm³, insbesondere bevorzugt 2,3 bis 3,5 g/cm³. Beispiele für geeignete Trägerkörper sind solche aus Magnesiumsilicat (Steatit), Quarz (SiO₂), Porzellan, Magnesiumoxid, Zinndioxid, Siliciumcarbid, Rutil, Tonerde (Al₂O₃), Zirkoniumsilikat, Aluminiumsilikat, Cersilicat oder aus Mischungen dieser Trägermaterialien. Besonders bevorzugt sind Steatit-Trägerkörper. Es wurde gefunden, dass carbidhaltige oder gesinterte Eisenoxid-Trägerkörper schlechtere Ergebnisse liefern.

Als inerte Trägerkörper werden Hohlzylinder eingesetzt. Nach einer weiter bevorzugten Ausführungsform weisen die verwendeten Trägerkörper-Hohlzylinder eine der folgenden Größen auf (Außendurchmesser x Höhe x Innendurchmesser): 6 x 5 x 3, 6 x 5 x 4, 6 x 4 x 4, 6 x 4 x 3, 5 x 5 x 3, 4 x 4 x 2, 4 x 4 x 1,5, 5 x 4 x 3, 5 x 5 x 2,5, 5 x 3 x 3, 4 x 3 x 1,5, 4 x 3 x 2, 3 x 3 x 1,5, 3 x 4 x 1,5 jeweils in mm.

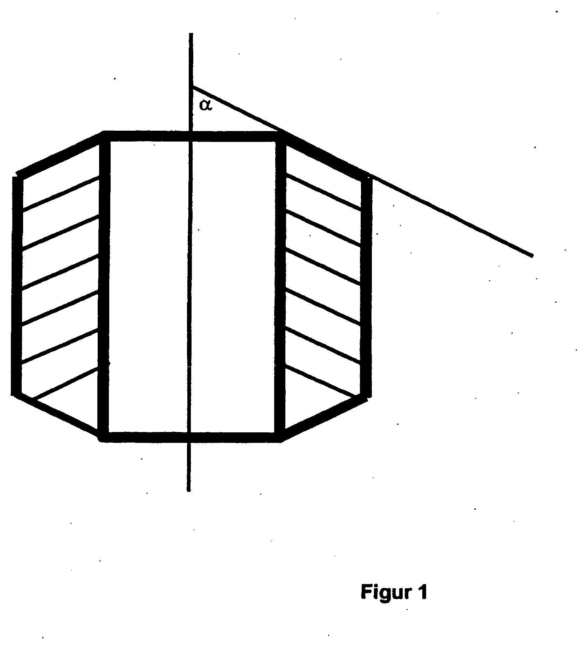

Weiterhin wurde überraschend gefunden, dass besonders gute Ergebnisse mit dem erfindungsgemäßen Katalysator bei der Oxidation von Methanol zu Formaldehyd erhalten werden können, wenn die Trägerkörper-Hohlzylinder mindestens einen, vorzugsweise genau einen zentralen Durchtrittskanal aufweisen und die Stirnseiten (Austrittsseiten des Durchtrittskanals) (nur) nach außen abgeflachte Kanten bzw. in einem spitzen Winkel (insbesondere 10-90°, weiter bevorzugt 30-85°, noch weiter bevorzugt 40-80°) zur Achse des Durchtrittskanals verlaufende Stirnflächen aufweisen, siehe Fig.1. Es können auch Formkörper gemäß der EP 1 127 618 A1 oder der WO 2005/037427 A1 verwendet werden.

Die vorstehend beschriebenen bevorzugten Trägerkörper in Form eines Zylinders mit Durchtrittskanal und stirnseitig nach außen abgeflachten Kanten bieten folgende Vorteile:
1. Die Formkörper bilden beim Beladen des Reaktors nicht so leicht lokal geordnete dichte Packungen sondern sind eher unregelmäßig angeordnet, wobei im Gasstrom durch das Katalysatorbett mehr Turbulenzen entstehen, welche einer leicht einsetzenden Überhitzung insbesondere bei der Oxidation von Methanol zu Formaldehyd entgegenwirken. Die Verringerung der Überhitzung führt auch zu einer Verlängerung der Lebensdauer des Katalysators.
2. Bei der Herstellung des Katalysators im bevorzugten Fließbettcoater besteht weniger Bruch an den Kanten als bei Verwendung von zylindrischen Körpern ohne abgeflachte Kanten.
3. Beim Füllen des Katalysators verkanten die Träger mit abgeflachten Kanten weniger als bei Verwendung von zylindrischen Körpern ohne abgeflachte Kanten. Dies gilt insbesondere für den Fall, dass der Wert von Reaktionsdurchmesser zu Katalysatordurchmesser zwischen etwa 2 und 5 liegt. Das Verkanten der Partikel führt zu sogenannten Fülllücken.

Auf den inerten Trägerkörper ist mindestens eine Schicht (Schale) einer Beschichtungszusammensetzung aufgebracht, die zunächst als aktive Komponente a) mindestens eine Molybdänverbindung und mindestens eine Eisenverbindung oder mindestens eine Fe- und Mohaltige Verbindung enthält, wobei das molare Verhältnis von Mo:Fe vorzugsweise zwischen etwa 1:1 und 5:1 liegt. Bevorzugt werden Oxide von Molybdän bzw. Eisen und/oder deren Mischoxide (wie Fe₂(MoO₄)₃) oder Verbindungen, die in die entsprechenden Oxide bzw. Mischoxide überführt werden können, wie beispielsweise Acetate, Oxalate, Acetylacetonate, Citrate, Nitrate, Chloride, Phosphate, Sulfate oder Ammoniumverbindungen des Eisens bzw. Molybdäns, eingesetzt. Nach einer besonders bevorzugten erfindungsgemäßen Ausführungsform wird ein Eisenmolybdän-Mischoxid (Fe₂(MoO₄)₃) als eisenhaltige Verbindung und MoO₃ als molybdänhaltige Verbindung verwendet. Daneben können in der vorstehenden Komponente a) auch gegebenenfalls weitere metallische bzw. metalloxidische Komponenten oder Verbindungen, die in die entsprechenden Oxide bzw. Mischoxide überführt werden können, enthalten sein, die dem Fachmann im Hinblick auf die Verwendung des Katalysators zur Oxidation von Methanol zu Formaldehyd geläufig sind. Nicht beschränkende Beispiele sind die Lanthanidenmetalle bzw. deren Oxide. Bevorzugt sind Titan, Antimon, Zinn, Nickel, Cer, Aluminium, Calcium, Magnesium, Chrom, Vanadium, Niob, Silber und/oder Mangan, die Fe und Mo auch teilweise ersetzen können.

Erfindungsgemäß umfasst die Beschichtung des erfindungsgemäßen Katalysators (vor dem Entfernen der organischen Anteile) mindestens einen organischen Binder (Komponente b)). Dieser Binder verbessert die Abriebfestigkeit der Beschichtung und trägt zu der guten Haftung der Beschichtung an dem inerten Trägerkörper bei. Zudem wird bei Verwendung eines organischen Binders wie hierin definiert auch ein besonders geringer Sprühverlust und somit eine besonders hohe Aufbringausbeute bei der Beschichtung der inerten Trägerkörper im Fließbettverfahren (Fließbettcoater) erzielt. Zusätzlich trägt er zur Ausbildung vorteilhafter Poren nach dem Entfernen der organischen Anteile der Beschichtungszusammensetzung bei. Es wurde nun gefunden, dass besonders vorteilhafte Ergebnisse mit Copolymeren, ausgewählt aus Vinylacetat/Vinyllaurat, Vinylacetat/Ethylen, Vinylacetat/Acrylat, Vinylacetat/Maleat, oder Styrol/Acrylat als organische Binder erhalten werden. Solche Copolymere sind dem Fachmann als solche geläufig, können nach Standardverfahren hergestellt werden und sind kommerziell erhältlich. Geeignete Dispersionen weisen vorzugsweise einen Feststoffgehalt im Bereich von 20 bis 80 Gew.-%, bevorzugt 40 - 60 Gew.-% auf. Geeignete Bindemittel umfassen z.B. Bindemitteldispersionen worin das Bindemittel ein Copolymer eines alpha-Olefins und eines Vinyl-C₂-C₄-Carboxylats ist, dessen Vinyl-C₂-C₄-Carboxylatgehalt wenigstens 62 Mol-% beträgt wie beispielsweise in der WO 2005/011862 genannt.

Daneben enthält die BeschichtungsZusammensetzung der erfindungsgemäßen Beschichtungskatalysatoren mindestens eine weitere Komponente (c)) ausgewählt aus der Gruppe bestehend aus SiO₂-Sol oder dessen Vorläufer, Al₂O₃-Sol oder dessen Vorläufer, ZrO₂-Sol oder dessen Vorlaufer, TiO₂-Sol oder dessen Vorläufer, Wasserglas, MgO, Zement, monomeres, oligomeres oder polymeres Silan, Alkoxysilan, Aryloxysilan, Acryloxysilan, Aminosilan, Siloxan oder Silanole. Diese Verbindungen sind dem Fachmann als solche geläufig, können nach Standardverfahren hergestellt werden und sind kommerziell erhältlich.

Unter den besonders bevorzugten Komponenten sind hier die anorganischen Sole, insbesondere TiO₂-Sole, Ceroxid-Sole und ZrO₂-Sole und zu nennen, da hier besonders gute Abriebfestigkeiten und zugleich hohe Aktivitäten der fertigen Katalysatoren bei der Oxidation von Methanol zu Formaldehyd erhalten wurden.

Je nach Sol liegt der Feststoffgehalt zwischen. 10 und 50 Gew.-%, beispielsweise sind SiO₂-Sole mit 20 - 40 Gew.-%, ZrO₂-Sole mit 10 - 20 Gew.-%, CeO₂-Sole mit 15 - 25 Gew.-% Feststoffgehalt und TiO₂-Sole mit 10 - 20 Gew.-% Feststoffgehalt bevorzugt. TiO₂-Sole sind besonders bevorzugt.

Es wurde unerwartet gefunden, dass die vorstehenden Komponenten b) und c) bei der Erzeugung einer optimalen Abriebfestigkeit und Porosimetrie des Katalysators zusammenwirken. Durch die erfindungsgemäß eingesetzte Haftvermittlerkombination (Kombination aus der mindestens einen Komponente b) und mindestens einer Komponente c)) wird sowohl ein Haften und vorteilhaftes Aufbringen der Beschichtungszusammensetzung bzw. der aktiven Masse (Komponente a)) auf dem Formkörper, insbesondere im bevorzugten Fließbettverfahren, als auch eine offenporige Struktur und gute Haftung der aktiven Masse nach dem Tempern bzw. Calcinieren des Katalysators bereitgestellt.

Nach einer bevorzugten erfindungsgemäßen Ausführungsform liegt das integrale Porenvolumen (bestimmt mit Hg-Porosimetrie, DIN 66133) zwischen etwa 100 - 800 mm³/g, bevorzugt zwischen etwa 200 und 700 mm³/g besonders bevorzugt zwischen etwa 250 und 600 mm³ / g. Der nach dieser Methode ermittelte mittlere Porenradius liegt vorzugsweise zwischen etwa 50 und 1000 nm, bevorzugt zwischen etwa 100 und 700 nm, besonders bevorzugt zwischen etwa 150 und 500 nm.

Weiterhin hat sich überraschend herausgestellt, dass der erfindungsgemäße ungetemperte Katalysator (Vorläufer-Katalysator), d.h. vor dem Entfernen der organischen Anteile der Beschichtungszusammensetzung eine stark verbesserte Transport- und Füllstabilität aufweist. Ein Aspekt der vorliegenden Erfindung betrifft somit auch die Verwendung eines erfindungsgemäßen Katalysators vor dem Entfernen der organischen Anteile der Beschichtungszusammensetzung (d.h. als Vorläufer-Katalysator) für den Transport von dem Produktionsort des Katalysators (bzw. Vorläufer-Katalysators) zum Einsatzort des Katalysators, insbesondere bei der Oxidation von Methanol zu Formaldehyd. Ein weiterer Aspekt betrifft die Verwendung eines solchen erfindungsgemäßen Katalysators vor dem Entfernen der organischen Anteile der Beschichtungszusammensetzung (d.h. als Vorläufer-Katalysator) zum Befüllen eines Reaktors, insbesondere zur Oxidation von Methanol zu Formaldehyd.

Nach einer bevorzugten erfindungsgemäßen Ausführungsform wird als Komponente c) mindestens ein Sol mit einer Partikelgröße von 1 bis 100 nm, vorzugsweise 2 bis 50 nm, besonders bevorzugt < 40 nm eingesetzt. Die Bestimmung dieser Partikelgrößen erfolgte nach ASTM B822-97. Alternativ kann auch ISO 13320-1 verwendet werden.

Besonders bevorzugt weist die Komponente c) eine kleinere mittlere Teilchengröße auf als die Komponente(n) a).

Besonders bevorzugt wird hierbei, dass Komponente c) ein ZrO₂-Sol, ein CeO₂-Sol, ein TiO₂-Sol oder eine Mischung aus zwei oder aller drei der vorstehenden Komponenten umfasst. Weiterhin wurde überraschend gefunden, dass besonders gute Abriebfestigkeiten mit einem TiO₂-Sol erhalten werden, das mit Salpetersäure oder mit Zitronensäure stabilisiert worden ist. Des Weiteren wurden gute Abriebfestigkeiten mit einem CeO₂-Sol, das mit Essigsäure stabilisiert ist bzw. einer Mischung aus dem TiO₂-Sol und dem CeO₂-Sol gefunden. Besonders vorteilhaft ist dabei, wenn das TiO₂-Sol im Überschuss gegenüber dem CeO₂-Sol vorliegt. Des Weiteren wurden gute Abriebfestigkeiten mit einem ZrO₂-Sol gefunden, das mit Acetat stabilisiert worden ist.

Neben den in einem Lösungsmittel bereitgestellten solen können auch sogenannte Aerosole eingesetzt werden, d.h. Metalloxide, die als Feststoff mit sehr hohen spezifischer Oberfläche bereitgestellt werden. Derartige feinverteilte Feststoffe lassen sich beispielsweise durch Flammhydrolyse darstellen, wie beispielsweise SiO₂ durch Hydrolyse von SiCl₄ in der H₂/O₂-Flamme. Durch vergleichbare verfahren lassen sich auch die anderen der oben genannten Oxide in feinverteilter Form darstellen. Die Verwendung von in einem Lösungsmittel bereitgestellten Solen ist jedoch bevorzugt, da die Verwendung dieser Sole zu Katalysatoren die eine besonders hohe Abriebsfestigkeit zeigen. Ferner werden bei Verwendung von Solen in Lösungsmitteln besonders geringe Sprühverluste beim Beschichten der Trägerkörper erreicht.

Erfindungsgemäß liegt der Feststoffanteil der Komponente c) (also z.B. ZrO₂ oder TiO₂) nach der Entfernung der organischen Anteile der Komponenten b) bzw. c), insbesondere nach dem Calcinieren bzw. Tempern des Katalysators (d.h. im fertigen Katalysator), zwischen 0,01 und 30 Gew.-%, bevorzugt zwischen 0,05 und 20 Gew.-%, weiter bevorzugt zwischen 0,1 und 10 Gew.-%, bezogen auf die katalytisch aktive BeschichtungsZusammensetzung, d.h. das Material der Schale. Die entsprechenden Einsatzmengen an Komponente c) können somit vom Fachmann leicht je nach der verwendeten Komponente c) und Komponente a) ermittelt werden.

weiterhin wird erfindungsgemäss bevorzugt, dass der Feststoffanteil der Komponente b), zwischen 5 und 30 Gew.-%, bevorzugt zwischen etwa 8 und 25 Gew.-% liegt, bezogen auf die katalytisch aktive Beschichtungszusammensetzung, d.h. das Material der Schale. Diese Angaben beziehen sich natürlich auf den Zustand vor

dem Entfernen der organischen Anteile der Beschichtungszusammensetzung.

Nach einer weiteren bevorzugten Ausführungsform der Erfindung liegt der Aktivmassenanteil (Komponente (a) / (Komponente (a) + Inertträger)) zwischen etwa 3 und 60 Gew.-%, bevorzugt zwischen etwa 3 und 50 Gew.-%, weiter bevorzugt zwischen etwa 5 und 40 Gew.-%, bezogen auf die Masse von Inertträger und katalytisch aktiver Masse. Bei den üblicherweise verwendeten Geometrien der inerten Trägerkörper ergibt sich eine erfindungsgemäß bevorzugte Dicke der Beschichtung auf dem inerten Trägerkörper zwischen etwa 30 und 1000 µm, bevorzugt zwischen etwa 100 und 700 µm.

Nach einer bevorzugten erfindungsgemäßen Ausführungsform liegt bei dem erfindungsgemäßen Katalysator nach dem Entfernen der organischen Anteile der Beschichtungszusammensetzung das auf die Aktivmasse bezogene integrale Porenvolumen (nach DIN 66133) bei mehr als etwa 0,15 ml/g/g_{Aktivmasse}, bevorzugt bei mehr als 0,2 ml/g_{Aktivmasse}, insbesondere bei mehr als 0,25 ml/g_{Aktivmasse}.

Ein weiterer Aspekt der vorliegenden Erfindung betrifft ein Verfahren zur Herstellung eines erfindungsgemäßen Beschichtungskatalysators, insbesondere zur Oxidation von Methanol zu Formaldehyd, umfassend die folgenden Schritte:
a) Bereitstellen eines inerten, vorzugsweise im wesentlichen unporösen Trägerkörpers,
b) Herstellen einer wässrigen Suspension, enthaltend die Komponenten a), b), c) wie oben definiert,
c) Aufbringen der wässrigen Suspension gemäß Schritt b) auf den inerten Trägerkörper, vorzugsweise im Fließbettverfahren. In einem ersten Schritt wird ein inerter, vorzugsweise im Wesentlichen unporöser Trägerkörper wie vorstehend beschrieben, bereitgestellt.

Weiterhin wird eine wässrige Suspension, enthaltend die Komponenten a), b), c), wie vorstehend beschrieben, bereitgestellt. Hierbei hat es sich erfindungsgemäß als besonders vorteilhaft herausgestellt, dass alle Komponenten a), b) und c) in Teilchenform, entweder suspendiert oder dispergiert und nicht in gelöster Form vorliegen. Der Feststoffgehalt in der Suspension / Dispersion liegt bevorzugt zwischen 5 und 40 Gew.-%, besonders bevorzugt zwischen 10 und 30 Gew.-%.

Bei dem erfindungsgemäßen Verfahren wird es bevorzugt, dass die bereitgestellte wässrige Suspension mittels eines Fließbettverfahrens auf den inerten Trägerkörper aufgebracht wird. Dabei wird die Verwendung eines Fließbettcoaters bevorzugt, wie er beispielsweise in der DE-A-12 80 756, der DE-A-197 09 589, der DE 40 06 935 A1, der DE 103 44 845 A1 oder der WO 2005/030388 beschrieben ist. Es hat sich erfindungsgemäß gezeigt, dass die wässrige Suspension mit der Beschichtungszusammensetzung mittels eines Fließbettverfahrens, wie vorstehend ausgeführt, besonders gleichmäßig und gut haftend sowie mit unerwartet geringen Sprühverlusten auf den unporösen Trägerkörper aufgebracht werden kann.

Weiterhin hat es sich als vorteilhaft herausgestellt, dass der pH-Wert der Suspension dem Stabilitätsbereich der Komponente b) und Komponente c) angepasst wird. Es hat sich gezeigt, dass hierzu allgemein ein pH-Wert der auf die Trägerkörper aufzubringenden Suspension zwischen etwa 1 und 5 von Vorteil ist. Es hat sich auch gezeigt, dass ein pH-Wert von etwa 3 - 5 im Falle der Verwendung eines acetatstabilisierten ZrO₂-Sols bzw. ein pH-Wert zwischen 1 und 5, vorzugsweise zwischen 1 und 3 im Falle der Verwendung eines mit Salpetersäure stabilisierten TiO₂-Sols von Vorteil ist. Im Falle des mit Essigsäure stabilisierten CeO₂-Sols ist ein pH Wert von 2 - 4 besonders vorteilhaft.

Weiterhin hat sich gezeigt, dass besonders günstige Ergebnisse erhalten werden, wenn die Komponente a) vor dem Auftragen auf den inerten Trägerkörper nicht auf über 200°C erhitzt wird, insbesondere nicht getempert bzw. calciniert wird.

Es ist jedoch gemäß einer Ausführungsform möglich, das Aktivmaterial (Komponente a) vor dem Auftragen auf den inerten Trägerkörper zu calcinieren, um beispielsweise seine Aktivität einzustellen. Es ist jedoch bevorzugt, den Katalysator in einer Form in den Reaktor einzufüllen, in welchem die Schale des Katalysators das uncalcinierte oder niedrig calcinierte Material sowie das organische Bindemittel enthält. Die Calcinierung des Aktivmaterials kann dann gleichzeitig mit dem Abbrennen des organischen Bindemittel erfolgen.

Nach einer weiteren bevorzugten erfindungsgemäßen Ausführungsform wird die wässrige Suspension auf die inerten Trägerkörper im Fließbettverfahren bei einer Temperatur von weniger als 100°C, insbesondere bei weniger als 80°C, weiter bevorzugt weniger als 70°C aufgebracht.

Bevorzugt weisen die Teilchen der Komponenten a), b) und c) in der wässrigen Suspension einen D₉₀-Wert der Teilchengröße von weniger als 50 µm, bevorzugt weniger als 30 µm auf. Die Einhaltung dieser Teilchengröße trägt zu einer besonders gleichmäßigen und abriebfesten Beschichtung auf den inerten Trägerkörpern bei und führt zu geringen Sprühverlusten während des Beschichtungsvorgangs. Sofern nicht ohnehin für die Komponenten a), b) und c) Materialien eingesetzt werden, die die vorstehende Teilchengröße aufweisen, kann diese durch herkömmliches Mahlen bzw. Zerkleinern vor, während oder nach der Herstellung der wässrigen Suspension eingestellt werden.

Nach einer weiteren bevorzugten Ausführungsform wird nach dem Aufbringen der Beschichtungszusammensetzung auf den inerten Trägerkörper der beschichtete Trägerkörper thermisch behandelt. Allgemein kann jede dem Fachmann geläufige Temperatur und Zeitdauer zur Calcinierung bzw. zum Tempern verwendet werden. In vielen Fallen wird eine Temperatur zwischen 200 und 600 °C, insbesondere 200 und 550 °C vorteilhaft sein. Die Dauer der Calcinierung bzw. des Temperns wird vorzugsweise zwischen 0,5 und 20 h, insbesondere zwischen 1 und 15 h liegen. Nach einer bevorzugten Ausführungsform wird der beschichtete Trägerkörper in einen geeigneten Wärmeschrank gegeben, z.B. einen Hordenofen mit Blechen, die den beschichteten Trägerkörper als Partikelschüttung mit einer bevorzugten Schüttungshöhe von 1 - 5 cm, besonders bevorzugt von 1 - 3 cm enthält. Der Wärmeschrank wird dann bevorzugt mit konstanter Rate in einem Zeitraum von 1 h bis 20 h, bevorzugt 5 h bis 15 h von Raumtemperatur auf eine Temperatur T1 von bevorzugt zwischen 130 °C bis 350 °C, insbesondere bevorzugt 200 bis 300 °C erhitzt. Die Aufheizrate für den vorgehend beschriebenen Prozessschritt wird bevorzugt im Bereich von 0,1 bis 5 °C/min, besonders bevorzugt 0,2 bis 1°C/min gewählt. Die Temperatur T1 wird dann bevorzugt für die Dauer von 1 h bis 5 h konstant gehalten. Anschließend wird die Temperatur mit vorzugsweise konstanter Rate in einem Zeitraum von 1 h bis 10 h, bevorzugt von 1 h bis 5 h ausgehend von der Temperatur T1 auf eine Endtemperatur T2 zwischen 300 bis 600 °C, bevorzugt 350 bis 550 °C, besonders bevorzugt 400 °C bis 550 °C erhitzt. Die Aufheizrate für den vorgehend beschriebenen Prozessschritt wird bevorzugt im Bereich von 0,1 bis 10 °C/min, besonders bevorzugt 2 bis 5 °C/min gewählt. Die Temperatur T2 wird für eine Zeitdauer von 1 - 10 h, bevorzugt von 2 - 5 h gehalten und der Wärmeschrank dann abgekühlt. Bevorzugt wird eine Abkühlrate von 1 - 10 °C / min, besonders bevorzugt von 2 - 8 °C / min gewählt. Während der thermischen Behandlung kann es vorteilhaft sein, den Gasraum des Wärmeschranks mit Luft oder einer Mischung zeitlich unterschiedlicher Zusammensetzung aus Luft, Stickstoff und gegebenenfalls Wasserdampf zu durchströmen.

Die gemäß der vorstehenden Beschreibung als Komponente b) bevorzugten organischen Binder weisen den Vorteil auf, dass sie bereits bei den vorstehenden relativ niedrigen Beschichtungstemperaturen im Fließbettcoater sehr gut zur Haftung und Abriebfestigkeit der Beschichtungszusammensetzung beitragen, und gleichzeitig bei den zum Erhitzen bzw. Calcinieren des Beschichtungskatalysators bevorzugten Temperaturen nach dem Aufbringen der Beschichtung auf den inerten Trägerkörper entfernt werden können, ohne die Haftung und Abriebfestigkeit der Beschichtung zu beeinträchtigen, um zu einem vorteilhaften Porengefüge beizutragen.

Nach einer bevorzugten Ausführungsform der Erfindung erfolgt das vorstehend beschriebene Erhitzen nach dem Aufbringen der Beschichtung auf den inerten Trägerkörper in einer Atmosphäre aus Luft, Inertgas und/oder Wasserdampf. Soweit das Erhitzen in Anwesenheit von Wasserdampf erfolgt, wird ein Wasserdampfgehalt von mehr als etwa 10 Vol.-%, bevorzugt zwischen etwa 20 und 60 Vol.-% der verwendeten Atmosphäre bevorzugt.

Soweit, wie vorstehend ausgeführt, nach einem bevorzugten Aspekt der vorliegenden Erfindung der erfindungsgemäße Beschichtungskatalysator vor dem Entfernen der organischen Anteile der Beschichtungszusammensetzung zum Befüllen des Reaktors selbst verwendet wird, wurde im Rahmen der vorliegenden Erfindung auch gefunden, dass die Entfernung der organischen Anteile der Beschichtungszusammensetzung (mittels Erhitzen bzw. Tempern oder Calcinieren des Katalysators) in vorteilhafter Weise auch direkt im Reaktor selbst, insbesondere in einem O₂- und N₂-haltigen Gasstrom, dem ggf. Wasserdampf zugesetzt sein kann. Die Bedingungen für das Calcinieren bzw. das Entfernen des organischen Bindemittels werden bevorzugt entsprechend den bereits für die Behandlung des Katalysators beschriebenen Bedingungen gewählt. Gemäß einer weiteren bevorzugten Ausführungsform kann das Calcinieren auch unter den für den fertigen Katalysator vorgesehenen Betriebsbedingungen, wie den Bedingungen der vorgesehenen Methanoloxidation zu Formaldehyd, stattfinden.

Ein weiterer erfindungsgemäßer Aspekt betrifft einen Katalysator, erhältlich nach dem vorstehenden Verfahren. Derartige Katalysatoren zeichnen sich gegenüber Katalysatoren des Standes der Technik durch eine erhöhte Abriebfestigkeit aus. Weiterhin ist durch eine vorteilhafte Porosimetrie nach dem Entfernen der organischen Anteile der Beschichtungszusammensetzung für die Katalysatoren eine hohe Aktivität und Formaldehydselektivität insbesondere bei der Oxidation von Methanol gewährleistet.

Somit betrifft ein weiterer erfindungsgemäßer Aspekt der vorliegenden Erfindung die Verwendung des vorstehend beschriebenen Beschichtungskatalysators zur Oxidation von Methanol zu Formaldehyd, insbesondere in einem Festbettverfahren. Ein geeignetes Verfahren ist beispielsweise aus der DE 103 61 517, der EP 0 001 570 und der US 3,852,361 bekannt. Es können auch andere dem Fachmann geläufige Verfahren zur Herstellung von Formaldehyd verwendet werden. Jedoch sind grundsätzlich auch andere Anwendungen des Katalysators, insbesondere bei partiellen (Gasphasen)-Oxidationen von Kohlenwasserstoffen nicht ausgeschlossen.

Die Gasphasenoxidation wird an sich in den für diese Reaktion bekannten Reaktoren bei üblichen Bedingungen durchgeführt. Bevorzugt sind Röhrenreaktoren, wobei die Röhren zur Wärmeabfuhr beispielsweise mit einem Salzbad oder einem temperaturbeständigen Öl gekühlt werden. Die Röhren haben bevorzugt einen Durchmesser im Bereich von 15 bis 30 mm, besonders bevorzugt von 20 - 25 mm, und eine Länge im Bereich von bevorzugt 80 bis 140 cm. In die Röhren wird der erfindungsgemäße Katalysator eingefüllt.

Ein weiterer erfindungsgemäßer Aspekt betrifft die Verwendung einer Kombination der vorstehend beschriebenen Komponenten b) und c) zur Erhöhung der Abriebfestigkeit eines Beschichtungskatalysators, insbesondere eines Katalysators zur Oxidation von Methanol zu Formaldehyd.

Figur 1 zeigt einen Querschnitt durch einen erfindungsgemäß bevorzugt verwendeten Trägerkörper mit stirnseitig nach außen abgeflachten Kanten. Der spitze Winkel α zwischen der Achse der zentralen Durchtrittsöffnung und den Stirnflächen liegt jeweils bei etwa 60 Grad.

### Begriffsdefinitionen:

a) **Aktivmassenanteil** = Masse Aktivmasse (Komponente a)) / (Masse Aktivmasse (Komponente a)) + Masse Trägerkörper)
b) **Feststoffanteil Komponente** b = Masse Feststoff im org. Bindemittel (Komponente b)) / (Masse Feststoff im org. Bindemittel (Komponente b)) + Masse Aktivmasse (Komponente a)))
c) **Feststoffanteil Komponente** c = Masse Feststoff in Komponente c) / (Masse Feststoff in Komponente c )+ Masse Aktivmasse (Komponente a)))

Erfindungsgemäß wurden folgende Bestimmungsmethoden verwendet:

### 1. Test zur Bestimmung der Haftfähigkeit der aktiven Masse (Abriebstest):

Die Haftfähigkeit der aktiven Masse wurde in einem Gerät der Fa. ERWEKA, Typ TAR 10 untersucht. Dazu wurde eine Menge von 50 g gecoatetem Katalysator bei einer Umdrehungsgeschwindigkeit von 75 Umdrehungen / min insgesamt 10 mal rotiert (= 10 Umdrehungen der Trommel). Es wurde danach der Abrieb als Verhältnis der losen, nicht mehr auf dem Trägerkörper gebunden Beschichtung zur insgesamt aufgetragenen Beschichtung bestimmt.

### 2. BET-Oberfläche:

Die Bestimmung erfolgt nach der BET-Methode gemäß DIN 66131; eine Veröffentlichung der BET-Methode findet sich auch in J. Am. Chem. Soc. 60, 309 (1938).

### 3. Porenradienverteilung:

Die Bestimmung der Porenradienverteilung erfolgte Quecksilberporosimetrie gemäß DIN 66133; maximaler Druck: 2.000 bar, Porosimeter 4000 (Firma Porotec, DE), nach Angaben des Herstellers.

### 4. Bestimmung der Teilchengrößen (Partikelgrößen) :

Die Bestimmung der Partikelgrößen erfolgte nach der Laserbeugungsmethode mit einem Fritsch Particle Sizer Analysette 22 Economy (Fa. Fritsch, DE) nach den Angaben des Herstellers, auch bezüglich der Probenvorbehandlung: die Probe wird in deionisiertem Wasser ohne Zusatz von Hilfsmitteln homogenisiert und 5 Minuten mit Ultraschall behandelt. Die angegebenen D-Werte sind auf das Probenvolumen bezogen.

Die Erfindung wird nun anhand der nachstehenden nicht beschränkenden Beispiele näher erläutert:

### Beispiel 1 (erfindungsgemäß):

Zur Herstellung des erfindungsgemäßen Katalysators mit einem Aktivmassenanteil (Komponente a)) von 20 Gew.-%, einem Feststoffanteil eines organischen Bindemittels (Komponente b)) von 20 Gew.-% und einem Feststoffanteil der anorganischen haftungsvermittelnden Komponente (c)) von 1 Gew.-%, wurden in einem Fließbettcoater 700 g Steatitkörper (Dichte 2,7 g/cm³) in Form von Hohlzylindern der Abmessung 5 x 5 x 2,5 mm mit einer Suspension, die wie folgt hergestellt wurde, beschichtet:

In einem Glasbehälter werden 1100 ml VE-Wasser vorgelegt. 184 g des Aktivmassenpulvers (Mischung aus Fe₂(MoO₄)₃ und MoO₃, hergestellt nach Beispiel 1 der EP 1 674 156 A1 (molares Verhältnis (Mo : Fe = 2,5) werden unter Rühren suspendiert. Zur besseren Homogenisierung wird die Suspension 3 min auf Stufe 6 mit dem Ultra - Turrax^{®} behandelt. Unter Rühren werden 9,22 g des ZrO₂-Sols (20 % Feststoffgehalt, acetatstabilisiert, Fa. Nyacol, Tradename: NYACOL^{®} Zirconia (Acetate)) dazugegeben. Der pH-Wert der Suspension wird mit einer 25 %-igen Ammoniaklösung auf 4 eingestellt. Zu der Suspension werden 92 g des organischen Bindemittels (50 %-ige Dispersion von Wasser und Vinylacetat / Ethylencopolymer, Vinnapas^{®} EP 65 W, Fa. Wacker) gegeben und die Suspension unter Rühren eine Stunde homogenisiert.

Die so erhaltene Beschichtungszusammensetzung wird in Form dünner Schichten bei einer Temperatur zwischen 60 - 80 °C auf die Steatitkörper im Fließbett aufgebracht. Die Schichtdicke des katalytisch aktiven Materials auf der Außenwand des Hohlzylinders betrug 300 µm.

Bei einer Temperatur von 400 °C bzw. 500 °C wurde für eine Zeitdauer von 2 Stunden ein Teil des gecoateten Katalysators thermisch in Luft behandelt und dabei die organischen Anteile der Beschichtungszusammensetzung (das organische Bindemittel) entfernt. Der Abrieb des bei 400 °C calcinierten Katalysators betrug 1,3 Gew.-%, der Abrieb des bei 500 °C calcinierten Katalysators betrug 3,1 Gew.-%. Der Abrieb des uncalcinierten Katalysators lag << 1 %.

Das mittels Hg-Porosimetrie gemessene integrale Porenvolumen beträgt für den bei 400 °C calcinierten Katalysator 0,42 ml/g, für den bei 500 °C calcinierten Katalysator 0,37 ml / g.

### Beispiel 2 (erfindungsgemäß):

Zur Herstellung des erfindungsgemäßen Katalysators mit einem Aktivmassenanteil (Komponente a)) von 20 Gew.-%, einem Feststoffanteil eines organischen Bindemittels (Komponente b)) von 20 Gew.-% und einem Feststoffanteil der anorganischen haftungsvermittelnden Komponente (c)) von 4,7 Gew.-%, wurden in einem sogenannten Fließbettcoater 700 g Steatitkörper (Dichte 2,7 g/cm³) in Form von Hohlzylindern der Abmessung 5 x 5 x 2,5 mm mit einer Suspension, die wie folgt hergestellt wurde, beschichtet:

In einem Glasbehälter werden 1100 ml VE-Wasser vorgelegt. 184 g des Aktivmassenpulvers (Mischung aus Fe₂(MoO₄)₃ und MoO₃ hergestellt nach Beispiel 1 der EP 1 674 156 A1 (molares Verhältnis (Mo : Fe = 2,5) werden unter Rühren suspendiert. Zur besseren Homogenisierung wird die Suspension 3 min auf Stufe 6 mit dem Ultra - Turrax^{®} behandelt. Unter Rühren werden 76 g des TiO₂-Sols (12 % Feststoffgehalt, mit Salpetersäure stabilisiert, Fa. Sachtleben, Tradename: Hombikat™ XXS 100) dazugegeben. Der pH-Wert der Suspension betrug 2,2. Zu der Suspension werden 92 g des organischen Bindemittels (50 %ige Dispersion von Wasser und Vinylacetat / Ethylencopolymer, Vinnapas^{®} EP 65 W, Fa. Wacker) gegeben und die Suspension unter Rühren eine Stunde homogenisiert.

Die so erhaltene Beschichtungszusammensetzung wird in Form dünner Schichten bei einer Temperatur zwischen 60 - 80 °C auf die Steatitkörper im Fließbett aufgebracht. Die Schichtdicke des katalytisch aktiven Materials betrug 305 µm.

Bei einer Temperatur von 400 °C bzw. 500 °C wurde für eine Zeitdauer von 2 Stunden ein Teil des gecoateten Katalysators thermisch in Luft behandelt und dabei die organischen Anteile der Beschichtungszusammensetzung (das organische Bindemittel) entfernt. Der Abrieb des bei 400 °C calcinierten Katalysators betrug 2,7 Gew.-%, der Abrieb des bei 500 °C calcinierten Katalysators betrug 5,3 Gew.-%. Der Abrieb des uncalcinierten Katalysators lag << 1 %.

Das mittels Hg-Porosimetrie gemessene integrale Porenvolumen beträgt für den bei 400 °C calcinierten Katalysator 0,44 ml/g, für den bei 500 °C calcinierten Katalysator 0,38 ml / g.

### Beispiel 3 (erfindungsgemäß):

Zur Herstellung des erfindungsgemäßen Katalysators mit einem Aktivmassenanteil (Komponente a)) von 26 Gew.-%, einem Feststoffanteil eines organischen Bindemittels (Komponente b)) von 20 Gew.-% und einem Feststoffanteil der anorganischen haftungsvermittelnden Komponente (c)) von 4,7 Gew.-%, wurden in einem Fließbettcoater 700 g Steatitkörper (Dichte 2,7 g/cm³) in Form von Hohlzylindern der Abmessung 5 x 5 x 2,5 mm mit einer Suspension, die wie folgt hergestellt wurde, beschichtet:

In einem Glasbehälter werden 1100 ml VE-Wasser vorgelegt. 245 g des Aktivmassenpulvers (Mischung aus Fe₂(MoO₄)₃ und MoO₃ hergestellt nach Beispiel 1 der EP 1 674 156 A1 (molares Verhältnis (Mo : Fe = 2,5)) werden unter Rühren suspendiert. Zur besseren Homogenisierung wird die Suspension 3 min auf Stufe 6 mit dem Ultra - Turrax^{®} behandelt. Unter Rühren werden 46 g des Ceroxid-Sols (20 % Feststoffgehalt, 3 % Essigsäure) dazugegeben. Der pH-Wert der Suspension wird mit einer 25 %-igen Ammoniaklösung auf 4 eingestellt. Zu der Suspension werden 122 g des organischen Bindemittels (50 %ige Dispersion von Wasser und Vinylacetat / Ethylencopolymer, Vinnapas^{®} EP 65 W, Fa. Wacker) gegeben und die Suspension unter Rühren eine Stunde homogenisiert.

Die so erhaltene Beschichtungszusammensetzung wird in Form dünner Schichten bei einer Temperatur zwischen 60 - 80 °C auf die Steatitkörper im Fließbett aufgebracht. Die Schichtdicke des katalytisch aktiven Materials betrug 424 µm.

Bei einer Temperatur von 400 °C bzw. 500 °C wurde für eine Zeitdauer von 2 Stunden ein Teil des gecoateten Katalysators thermisch in Luft behandelt und dabei die organischen Anteile der Beschichtungszusammensetzung (das organische Bindemittel) entfernt. Der Abrieb des bei 400 °C calcinierten Katalysators betrug 0,7 Gew.-%, der Abrieb des bei 500 °C calcinierten Katalysators betrug 1,3 Gew.-%. Der Abrieb des uncalcinierten Katalysators lag << 1 %.

Das mittels Hg-Porosimetrie gemessene integrale Porenvolumen beträgt für den bei 400 °C calcinierten Katalysator 0,41 ml/g, für den bei 500 °C calcinierten Katalysator 0,37 ml / g.

### Beispiel 4 (erfindungsgemäß):

Zur Herstellung des erfindungsgemäßen Katalysators mit einem Aktivmassenanteil (Komponente a)) von 20 Gew.-%, einem Feststoffanteil eines organischen Bindemittels (Komponente b)) von 20 Gew.-% und einem Feststoffanteil der anorganischen haftungsvermittelnden Komponente (c)) von 4,7 Gew.-% wurden in einem Fließbettcoater 700 g Steatitkörper (Dichte 2,7 g/cm³) in Form von Hohlzylindern der Abmessung 5 x 5 x 2,5 mm mit einer Suspension, die wie folgt hergestellt wurde, beschichtet:

In einem Glasbehälter werden 1100 ml VE-Wasser vorgelegt. 184 g des Aktivmassenpulvers (Mischung aus Fe₂(MoO₄)₃ und MoO₃ hergestellt nach Beispiel 1 der EP 1 674 156 A1 (molares Verhältnis (Mo : Fe = 2,5)) werden unter Rühren suspendiert. Zur besseren Homogenisierung wird die Suspension 3 min auf Stufe 6 mit dem Ultra - Turrax^{®} behandelt. Unter Rühren werden 46 g des SiO₂-Sols (GRACE Davidson, 30% Feststoffgehalt, Tradename: Ludox^{®} AS-30) dazugegeben. Der pH-Wert der Suspension wird mit einer 25 %-igen Ammoniaklösung auf 4 eingestellt. Zu der Suspension werden 92 g des organischen Bindemittels (50 %ige Dispersion von Wasser und Vinylacetat / Ethylencopolymer, Vinnapas^{®} EP 65 W, Fa. Wacker) gegeben und die Suspension unter Rühren eine Stunde homogenisiert.

Die so erhaltene Beschichtungszusammensetzung wird in Form dünner Schichten bei einer Temperatur zwischen 60 - 80 °C auf die Steatitkörper im Fließbett aufgebracht. Die Schichtdicke des katalytisch aktiven Materials betrug 300 µm.

Bei einer Temperatur von 400 °C bzw. 500 °C wurde für eine Zeitdauer von 2 Stunden ein Teil des gecoateten Katalysators thermisch in Luft behandelt und dabei die organischen Anteile der Beschichtungszusammensetzung (das organische Bindemittel) entfernt. Der Abrieb des bei 400 °C calcinierten Katalysators betrug 2,7 Gew.-%, der Abrieb des bei 500 °C calcinierten Katalysators betrug 4,2 Gew.-%. Der Abrieb des uncalcinierten Katalysators lag << 1 %.

Das mittels Hg-Porosimetrie gemessene integrale Porenvolumen beträgt für den bei 400 °C calcinierten Katalysator 0,39 ml/g, für den bei 500 °C calcinierten Katalysator 0,35 ml / g.

### Beispiel 5 (Vergleich):

Zur Herstellung des Vergleichskatalysators mit einem Aktivmassenanteil (Komponente a)) von 21 Gew.-%, und einem Feststoffanteil des organischen Bindemittels (Komponente b)) von 20 Gew.-% wurden in einem sogenannten Wirbelbettcoater 700 g Steatitkörper (Dichte 2,7 g/cm³) in Form von Hohlzylindern der Abmessung 5 x 5 x 2,5 mm mit einer Suspension, die wie folgt hergestellt wurde, beschichtet:

In einem Glasbehälter werden 1100 ml VE-Wasser vorgelegt. 184 g des Aktivmassenpulvers (Mischung aus Fe₂(MoO₄)₃ und MoO₃, hergestellt nach Beispiel 1 der EP 1 674 156 A1 (molares Verhältnis (Mo : Fe = 2,5)) werden unter Rühren suspendiert. Zur besseren Homogenisierung wird die Suspension 3 min auf Stufe 6 mit dem Ultra - Turrax^{®} behandelt. Der pH-Wert der Suspension wird mit einer 25 %-igen Ammoniaklösung auf 4 eingestellt. Zu der Suspension werden 92 g des organischen Bindemittels (50 %ige Dispersion von Wasser und Vinylacetat / Ethylencopolymer, Vinnapas^{®} EP 65 W, Fa. Wacker) gegeben und die Suspension unter Rühren eine Stunde homogenisiert.

Die so erhaltene Beschichtungszusammensetzung wird in Form dünner Schichten bei einer Temperatur zwischen 60 - 80 °C im Fließbett auf die Steatitkörper aufgebracht. Die Schichtdicke des katalytisch aktiven Materials betrug 312 µm.

Bei einer Temperatur von 400 °C bzw. 500 °C wurde für eine Zeitdauer von 2 Stunden ein Teil des gecoateten Katalysators thermisch behandelt und dabei das organische Bindemittel entfernt. Der Abrieb des bei 400 °C calcinierten Katalysators betrug 37,9 Gew.-%, der Abrieb des bei 500 °C calcinierten Katalysators betrug 38,3 Gew.-%. Der Abrieb des uncalcinierten Katalysators lag etwas unter 1 % und deutlich höher als bei den vorstehenden erfindungsgemäßen Katalysatoren.

Das mittels Hg-Porosimetrie gemessene integrale Porenvolumen beträgt für den bei 400 °C calcinierten Katalysator 0,46 ml/g, für den bei 500 °C calcinierten Katalysator 0,42 ml / g.

## Patentansprüche

1. Beschichtungskatalysator in ungetemperter Form, insbesondere zur Oxidation von Methanol zu Formaldehyd, der auf einem inerten, vorzugsweise im wesentlichen unporösen Trägerkörper in Form eines Hohlzylinders mindestens eine Beschichtung aufweist, die vor dem Entfernen der organischen Anteile der Komponenten b) bzw. c) enthält:
a) oxide oder in die entsprechenden Oxide überführbare Vorläuferverbindungen von Molybdän und Eisen, wobei das molare Verhältnis von Mo:Fe zwischen 1:1 und 5:1 liegt, sowie gegebenenfalls weitere metallische bzw. metalloxidische Komponenten oder in die entsprechenden Oxide überführbare Vorläuferverbindungen,
b) mindestens einen organischen Binder, wobei das organische Bindemittel ein Copolymer ausgewählt aus Vinylacetat/Vinyllaurat, Vinylacetat/Ethylen, Vinylacetat/Acrylat, Vinylacetat/Maleat, Styrol/Acrylat oder deren Gemischen ist
c) und mindestens eine weitere haftungsvermittelnde Komponente ausgewählt aus der Gruppe bestehend aus SiO₂-Sol oder dessen Vorläufer, Al₂O₃-Sol oder dessen Vorläufer, ZrO₂-Sol oder dessen Vorläufer, TiO₂-Sol oder dessen Vorläufer, CeO₂-Sol Wasserglas, MgO, Zement, monomeren, oligomeren oder polymeren Silanen, Alkoxysilanen, Aryloxysilanen, Acryloxysilanen, Aminosilanen, Siloxanen oder Silanolen.
wobei der Anteil der Komponente c) nach Entfernung der organischen Anteile der Komponenten b) und ggf. c) zwischen 0,01 und 30 Gew.-% beträgt, bezogen auf die katalytisch aktive Beschichtungszusammensetzung.

2. Beschichtungskatalysator nach einem der vorstehenden Ansprüche, **dadurch gekennzeichnet, dass** als Komponente c) mindestens ein Sol mit einer Partikelgröße von 1 bis 100 nm, vorzugsweise 2 bis 50 nm, weiter bevorzugt 3 - 40 nm eingesetzt wird.

3. Beschichtungskatalysator nach einem der vorstehenden Ansprüche, **dadurch gekennzeichnet, dass** die Komponente c) TiO₂-Sol oder zrO₂-Sol oder CeO₂-Sol oder eine Mischung von zwei oder aller drei genannten Komponenten umfasst.

4. Beschichtungskatalysator nach einem der vorstehenden Ansprüche, **dadurch gekennzeichnet, dass** der Feststoffanteil der Komponente c) nach Entfernung der organischen Anteile der Komponenten b) und c) und bezogen auf die katalytisch aktive Beschichtungszusammensetzung zwischen 0,05 und 20 Gew.-%, weiter bevorzugt zwischen 0,1 und 10 Gew.-% liegt.

5. Beschichtungskatalysator nach einem der vorstehenden Ansprüche, **dadurch gekennzeichnet, dass** der Feststoffanteil der Komponente b) bezogen auf die katalytisch aktive Beschichtungszusammensetzung und bezogen auf einen Zustand vor dem Entfernen der organischen Anteile der Beschichtungszusammensetzung zwischen 5 und 30 Gew.-%, bevorzugt zwischen 8 und 25 Gew.-% liegt.

6. Beschichtungskatalysator nach einem der vorstehenden Ansprüche, **dadurch gekennzeichnet, dass** der inerte Trägerkörper ein Zylinder mit Durchtrittskanal und stirnseitlich nach außen abgeflachten Kanten darstellt.

7. Beschichtungskatalysator nach einem der vorstehenden Ansprüche, **dadurch gekennzeichnet, dass** der Aktivmassenanteil zwischen etwa 3 und 60 Gew.-%, bevorzugt zwischen etwa 3 und 50 Gew.-%, weiter bevorzugt zwischen etwa 5 und 40 Gew.-% liegt.

8. Beschichtungskatalysator nach einem der vorstehenden Ansprüche, **dadurch gekennzeichnet, dass** die Dicke der Beschichtung auf dem inerten Träger zwischen etwa 30 und 1000 µm, bevorzugt zwischen etwa 100 und 700 µm liegt.

9. Beschichtungskatalysator nach einem der vorstehenden Ansprüche, **dadurch gekennzeichnet, dass** die weiteren metallischen bzw. metalloxidischen Komponenten der Komponente a) aus der Gruppe der Lanthaniden bzw. deren Oxiden und/oder Titan, Antimon, Zinn, Nickel, Chrom, Aluminium, Calcium, Magnesium, Vanadium, Silber und Mangan ausgewählt sind.

10. Beschichtungskatalysator nach einem der vorstehenden Ansprüche, **dadurch gekennzeichnet, dass** das integrale Porenvolumen der Beschichtung nach Entfernen der organischen Anteile der Komponenten b) bzw. c) größer als 0,2, insbesondere grösser als 0,25 ml/g liegt.

11. Verfahren zur Herstellung eines Beschichtungskatalysators gemäß einem der vorstehenden Ansprüche, insbesondere zur Oxidation von Methanol zu Formaldehyd, umfassend die folgenden Schritte:
a) Bereitstellen eines inerten, vorzugsweise im wesentlichen unporösen Trägerkörpers,
b) Herstellen einer wässrigen Suspension, enthaltend die Komponenten a), b), c) wie in den vorstehenden Ansprüchen definiert,
c) Aufbringung der wässrigen Suspension gemäß Schritt b) auf den inerten Trägerkörper im Fließbettverfahren.

12. Verfahren nach Anspruch 11, **dadurch gekennzeichnet, dass** das Aufbringen der Beschichtung im Fließbettverfahren bei weniger als 100 °C, vorzugsweise bei weniger als 80 °C, insbesondere bei weniger als 70 °C durchgeführt wird.

13. Verfahren nach einem der Ansprüche 11 oder 12, **dadurch gekennzeichnet, dass** die Komponenten a) und b) , vorzugsweise die Komponenten a), b) und c) in der wässrigen Suspension gemäß Schritt b) von Anspruch 11 als Suspension oder Dispersion und nicht in gelöster Form vorliegen.

14. Verfahren nach einem der Ansprüche 11 bis 13, **dadurch gekennzeichnet, dass** die Komponente a) vor dem Auftragen auf den inerten Trägerkörper nicht auf über 200 °C erhitzt wird, insbesondere nicht getempert bzw. calciniert wird.

15. Verfahren nach einem der Ansprüche 11 bis 14, **dadurch gekennzeichnet, dass** die wässrige Suspension eine Teilchengröße (D₉₀) von weniger als 50 µm, bevorzugt weniger als 30 µm aufweist.

16. Verfahren nach einem der Ansprüche 11 bis 15, **dadurch gekennzeichnet, dass** der Beschichtungskatalysator nach dem Aufbringen auf den inerten Trägerkörper auf eine Temperatur zwischen etwa 150 und 600 °C, bevorzugt zwischen 200 und 550 °C, weiter bevorzugt zwischen 250 und 500 °C erhitzt wird.

17. Verfahren nach einem der Ansprüche 11 bis 16, **dadurch gekennzeichnet, dass** das Erhitzen als Calcinierung bei einer Temperatur zwischen etwa 350 und 450 °C, bevorzugt bei etwa 400 °C erfolgt.

## Claims

1. Coated catalyst in untempered form, in particular for oxidizing methanol to formaldehyde, which has at least one coating on an inert, preferably essentially nonporous, support body in the form of a hollow cylinder, which comprises, before the removal of the organic fractions of components b) and c):
a) oxides, or precursor compounds convertible to the corresponding oxides, of molybdenum and iron, wherein the molar ratio of Mo:Fe is between 1:1 and 5:1, as well as optionally further metallic or metal oxide components, or precursor compounds convertible to the corresponding oxides,
b) at least one organic binder, wherein the organic binder is a copolymer selected from vinyl acetate/vinyl laurate, vinyl acetate/ethylene, vinyl acetate/acrylate, vinyl acetate/maleate, styrene/acrylate or mixtures thereof,
c) and at least one further adhesion-promoting component selected from the group consisting of SiO₂ sol or precursors thereof, Al₂O₃ sol or precursors thereof, ZrO₂ sol or precursors thereof, TiO₂ sol or precursors thereof, CeO₂ sol, waterglass, MgO, cement, monomeric, oligomeric or polymeric silanes, alkoxysilanes, aryloxysilanes, acryloxysilanes, aminosilanes, siloxanes or silanols.
wherein the content of component c) after removal of the organic fractions of components b) and optionally c) is between 0.01 und 30 wt.-%, based on the catalytically active coating composition.

2. Coated catalyst according to one of the previous claims, **characterized in that** at least one sol with a particle size of from 1 to 100 nm, preferably 2 to 50 nm, further preferably 3 - 40 nm, is used as component c).

3. Coated catalyst according to one of the previous claims, **characterized in that** component c) comprises TiO₂ sol or ZrO₂ sol or CeO₂ sol or a mixture of two or all three named components.

4. Coated catalyst according to one of the previous claims, **characterized in that** the solids content of component c), after removal of the organic fractions of components b) and c) and based on the catalytically active coating composition, is between 0.05 and 20 wt.-%, further preferably between 0.1 and 10 wt.-%.

5. Coated catalyst according to one of the previous claims, **characterized in that** the solids content of component b), based on the catalytically active coating composition and based on a state before removal of the organic fractions of the coating composition, is between 5 and 30 wt.-%, preferably between 8 and 25 wt.-%.

6. Coated catalyst according to one of the previous claims, **characterized in that** the inert support body comprises a cylinder with a passage channel and edges flattened on the outside at the ends.

7. Coated catalyst according to one of the previous claims, **characterized in that** the active composition content is between approximately 3 and 60 wt.-%, preferably between approximately 3 and 50 wt.-%, further preferably between approximately 5 and 40 wt.-%.

8. Coated catalyst according to one of the previous claims, **characterized in that** the thickness of the coating on the inert support is between approximately 30 and 1000 µm, preferably between approximately 100 and 700 µm.

9. Coated catalyst according to one of the previous claims, **characterized in that** the further metallic or metal oxide components of component a) are selected from the group of lanthanides or their oxides and/or titanium, antimony, tin, nickel, chromium, aluminium, calcium, magnesium, vanadium, silver and manganese.

10. Coated catalyst according to one of the previous claims, **characterized in that** the integral pore volume of the coating, after removal of the organic fractions of components b) and c), is greater than 0.2, in particular greater than 0.25 ml/g.

11. Method of preparing a coated catalyst according to one of the previous claims, in particular for oxidizing methanol to formaldehyde, comprising the following steps:
a) providing an inert, preferably essentially nonporous, support body,
b) preparing an aqueous suspension comprising components a), b), c) as defined in the previous claims,
c) applying the aqueous suspension according to step b) to the inert support body in a fluidized bed process.

12. Method according to claim 11, **characterized in that** the application of the coating in a fluidized bed process is carried out at less than 100°C, preferably at less than 80°C, in particular at less than 70°C.

13. Method according to one of claims 11 or 12, **characterized in that** components a) and b), preferably components a), b) and c), are present in the aqueous suspension according to step b) of claim 11 as a suspension or dispersion and not in dissolved form.

14. Method according to one of claims 11 to 13, **characterized in that** component a) is not heated to more than 200°C, and in particular is not tempered or calcined, before being applied to the inert support body.

15. Method according to one of claims 11 to 14, **characterized in that** the aqueous suspension has a particle size(D₉₀) of less than 50 µm, preferably less than 30 µm.

16. Method according to one of claims 11 to 15, **characterized in that** the coated catalyst is heated to a temperature between approximately 150 and 600°C, preferably between 200 and 550°C, further preferably between 250 and 500°C, after being applied to the inert support body.

17. Method according to one of claims 11 to 16, **characterized in that** the heating is carried out as a calcination at a temperature between approximately 350 und 450°C, preferably at approximately 400°C.

## Revendications

1. Catalyseur de revêtement sous forme non tempérée, notamment pour l'oxydation de méthanol en formaldéhyde, qui présente sur un corps de support inerte, de préférence essentiellement non poreux, sous la forme d'un cylindre creux au moins un revêtement qui contient avant l'élimination des portions organiques des composants b) et/ou c) :
a) des oxydes ou des composés précurseurs pouvant être transformés en les oxydes correspondants de molybdène et de fer, dans lequel le rapport molaire entre Mo/Fe se situe entre 1/1 et 5/1, ainsi qu'éventuellement d'autres composants métalliques ou d'oxydes métalliques ou composés précurseurs pouvant être transformés en les oxydes correspondants,
b) au moins un liant organique, dans lequel le liant organique est un copolymère sélectionné parmi l'acétate de vinyle/laurate de vinyle, l'acétate de vinyle/éthylène, l'acétate de vinyle/acrylate, l'acétate de vinyle/maléate, le styrène/acrylate ou leurs mélanges
c) et au moins un autre composant promoteur d'adhésion sélectionné parmi le groupe constitué de SiO₂-Sol ou ses précurseurs, Al₂O₃-Sol ou ses précurseurs, ZrO₂-Sol ou ses précurseurs, TiO₂-Sol ou ses précurseurs, CeO₂-Sol, du verre soluble, MgO, du ciment, des silanes, alcoxysilanes, aryloxysilanes, acryloxysilanes, aminosilanes, siloxanes ou silanols monomères, oligomères ou polymères,
dans lequel la proportion du composant c) après élimination des portions organiques des composants b) et éventuellement c) est comprise entre 0,01 et 30 % en poids par rapport à la composition de revêtement active du point de vue catalytique.

2. Catalyseur de revêtement selon l'une quelconque des revendications précédentes, **caractérisé en ce qu'**au moins un sol avec une taille particulaire de 1 à 100 nm, de préférence 2 à 50 nm, plus préférablement 3 à 40 nm, est utilisé en tant que composant c).

3. Catalyseur de revêtement selon l'une quelconque des revendications précédentes, **caractérisé en ce que** le composant c) comprend TiO₂-Sol ou ZrO₂-Sol ou CeO₂-Sol ou un mélange de deux ou de l'ensemble des trois composants cités.

4. Catalyseur de revêtement selon l'une quelconque des revendications précédentes, **caractérisé en ce que** la proportion de substances solides du composant c) après élimination des portions organiques des composants b) et c) et par rapport à la composition de revêtement active du point de vue catalytique se situe entre 0,05 et 20 % en poids, plus préférablement entre 0,1 et 10 % en poids.

5. Catalyseur de revêtement selon l'une quelconque des revendications précédentes, **caractérisé en ce que** la proportion de substances solides du composant b) par rapport à la composition de revêtement active du point de vue catalytique et par rapport à un état avant l'élimination des portions organiques de la composition de revêtement se situe entre 5 et 30 % en poids, de préférence entre 8 et 25 % en poids.

6. Catalyseur de revêtement selon l'une quelconque des revendications précédentes, **caractérisé en ce que** le corps de support inerte représente un cylindre avec canal de passage et des arêtes aplaties vers l'extérieur du côté frontal.

7. Catalyseur de revêtement selon l'une quelconque des revendications précédentes, **caractérisé en ce que** la proportion de masse active se situe entre environ 3 et 60 % en poids, de préférence entre environ 3 et 50 % en poids, plus préférablement entre environ 5 et 40 % en poids.

8. Catalyseur de revêtement selon l'une quelconque des revendications précédentes, **caractérisé en ce que** l'épaisseur du revêtement sur le support inerte se situe entre environ 30 et 1000 µm, de préférence entre environ 100 et 700 µm.

9. Catalyseur de revêtement selon l'une quelconque des revendications précédentes, **caractérisé en ce que** les autres composants métalliques ou d'oxydes métalliques du composant a) sont sélectionnés parmi le groupe des lanthanides ou de leurs oxydes et/ou du titane, de l'antimoine, de l'étain, du nickel, du chrome, de l'aluminium, du calcium, du magnésium, du vanadium, de l'argent et du manganèse.

10. Catalyseur de revêtement selon l'une quelconque des revendications précédentes, **caractérisé en ce que** le volume de pores intégral du revêtement après élimination des portions organiques des composants b) et/ou c) est supérieur à 0,2, notamment supérieur à 0,25 ml/g.

11. Procédé de fabrication d'un catalyseur de revêtement selon l'une quelconque des revendications précédentes, notamment pour l'oxydation de méthanol en formaldéhyde, comprenant les étapes suivantes :
a) mise à disposition d'un corps de support inerte, de préférence essentiellement non poreux,
b) fabrication d'une suspension aqueuse contenant les composants a), b), c) tels que définis dans les revendications précédentes,
c) application de la suspension aqueuse selon l'étape b) sur le corps de support inerte dans le procédé à lit fluidisé.

12. Procédé selon la revendication 11, **caractérisé en ce que** l'application du revêtement dans le procédé à lit fluidisé est effectuée à moins de 100°C, de préférence à moins de 80°C, notamment à moins de 70°C.

13. Procédé selon l'une quelconque des revendications 11 ou 12, **caractérisé en ce que** les composants a) et b), de préférence les composants a), b) et c) sont présents dans la suspension aqueuse selon l'étape b) de la revendication 11 en tant que suspension ou dispersion et non sous forme dissoute.

14. Procédé selon l'une quelconque des revendications 11 à 13, **caractérisé en ce que** le composant a) n'est pas chauffé à plus de 200°C, notamment n'est pas tempéré ou calciné, avant l'application sur le corps de support inerte.

15. Procédé selon l'une quelconque des revendications 11 à 14, **caractérisé en ce que** la suspension aqueuse présente une taille particulaire (D₉₀) inférieure à 50 µm, de préférence inférieure à 30 µm.

16. Procédé selon l'une quelconque des revendications 11 à 15, **caractérisé en ce que** le catalyseur de revêtement est chauffé après l'application sur le corps de support inerte à une température comprise entre environ 150 et 600°C, de préférence entre 200 et 550°C, plus préférablement entre 250 et 500°C.

17. Procédé selon l'une quelconque des revendications 11 à 16, **caractérisé en ce que** le chauffage s'effectue en tant que calcination à une température comprise entre environ 350 et 450°C, de préférence à environ 400°C.
